(19) Europäisches Patentamt

European Patent Office

Office européen des brevets

(11) Publication number: **0 359 195 B1**

(12) **EUROPEAN PATENT SPECIFICATION**

(45) Date of publication of patent specification: **15.12.93**   (51) Int. Cl.⁵: **A61K  9/54**, A61K 9/56, A61K 9/52, A61K 9/10

(21) Application number: **89116855.1**

(22) Date of filing: **12.09.89**

(54) **Controlled release therapeutic system for liquid pharmaceutical formulations.**

(30) Priority: **16.09.88 IT 2196188**

(43) Date of publication of application:
**21.03.90 Bulletin  90/12**

(45) Publication of the grant of the patent:
**15.12.93 Bulletin  93/50**

(84) Designated Contracting States:
**AT BE CH DE ES FR GB GR IT LI LU NL SE**

(56) References cited:
**WO-A-85/03000**
**WO-A-87/07833**
**WO-A-88/02253**
**US-A- 2 921 883**

(73) Proprietor: **RECORDATI S.A. CHEMICAL and PHARMACEUTICAL COMPANY**
**Corso S. Gottardo 54**
**CH-6830 Chiasso(CH)**

(72) Inventor: **Santus, Giancarlo**
**Via Zuara, 8**
**I-20146 Milan(IT)**
Inventor: **Golzi, Roberto**
**Via Enrico Toti, 28**
**I-26013 Crema (Prov. of Cremona)(IT)**

(74) Representative: **Passini, Angelo et al**
**NOTARBARTOLO & GERVASI S. R. L.**
**Viale Bianca Maria, 33**
**I-20122 Milano (IT)**

Rank Xerox (UK) Business Services
(3. 10 / 3.6 / 3.3. 1)

## Description

An important aspect of the administration of drugs in conventional forms is the fluctuation between high and low plasma concentration of the drug in the period between the assumption of two successive doses. In fact, it can happen that, if the drugs are too rapidly adsorbed, they can supply too high plasma levels, leading to undesiderable and even toxic side effects. On the other hand, those drugs possessing a short half-life are eliminated too rapidly and require therefore frequent administrations. In both cases the patient must be careful because particular attention and constancy in the administration is required during the therapy and said conditions can not always be easily obtained.

Pharmaceutical research has therefore made many many efforts in the attempt to formulate pharmaceutical preparations apt to protract in time the permanence of the active principle in the organism at optimum plasmatic levels, reducing the number of administrations and thus improving the response of the patient to the treatment.

The preparation of pharmaceutical composition apt to supply a gradual and controlled release in time of the active ingredient is well known in the pharmaceutical technology field.

Systems are known comprising tablets, capsules, microcapsules, microspheres and formulations in general in which the active principle is released gradually by means of various mechanisms.

It is also known that the oral administration is by far the most frequently employed in the therapy and the most appreciated by the patient, particularly when repeated assumptions are requested. Among the formulations for oral administration, the liquid ones present the advantage of beeing easily adaptable to the patient in the dosage and better accepted by the patients, particularly by children and elderly people. For this reason a liquid controlled release formulation is to be considered more advantageous with respect to a corresponding controlled release solid formulation.

In the preparation of controlled release liquid pharmaceutical compositions several problems may be encountered.

One of them consists in the fact that it is necessary that the actual controlled release forms be of such a size to be easily suspended and kept in suspension in the liquid vehicle; furthermore poor or non-homogenous distribution inside the vehicle is to be avoided as far as possible, and it is also preferable to avoid the unpalatabe sensation which one can have when ingesting a suspension containing a solid in coarse particles (sand effect). This can be obtained by suitably reducing the size of the forms to be suspended, but, on the other hand, the use of reduced size particles, increasing the surface area available for diffusion, makes it difficult to obtain a costant and protracted control of the release, as in the forms having larger dimensions.

A further problem is the difficulty of obtaining controlled release liquid preparations apt to maintain for long times the release characteristics of the pharmaceutical substances contained. The solution of said problems is commercially and therapeutically important both for the necessity of having ready to use liquid dosage formulations as far as possible stable in time, and for having liquid formulations which, prepared at the moment of use, will remain stable for a long time once the dispersion of the therapeutic agent in the dosage liquid has taken place.

All the above mentioned difficulties may explain why, as far as we know, only few controlled release liquid systems are known up to now, and among them only one is actually commercially available.

As examples of known controlled release liquid formulations, we may cite the ones described in the following patents: BE 903540, WO85/03000, WO 87/07833, US 4221778 and US4717713.

The necessity is felt therefore of a system which allows the administration of substances in a liquid oral dosage form,in such a way that the release in time of said substances be effected in the best possible way according to preestablished schedules. Such a system should be adjustable according to the peculiar therapeutic characteristics of the active principle to be administered, should possess homogeneity characteristics such as to allow a correct dosage, and should keep unchanged its characteristics in the selected vehicle for a long period of time even after the beginning of the treatment.

We have now found and it is an object of the present invention, that it is possible to obtain a controlled release therapeutic system for pharmaceutical liquid formulations having the above mentioned characteristics.

Such a system consists essentially of microgranules containing the active principle, particularly theophylline and its pharmaceutical acceptable salts, as well as suitable excipients, coated by several alternated layers of suitably selected materials, in a vehicle containing an immediately available dose of the active principle contained in the microgranules.

In the present document, when speaking of theophylline we intend to include always all its pharmacologically acceptable salts.

2

In order to obtain a system having the required characteristics, it is necessary to obtain microgranules having a surface apt to allow a uniform formation of film which can guarantee a constant release.

For this reason, it is necessary that the active principle be first granulated and that the granules have a homogenously smooth surface. Furthermore, in order to obtain the optimum suspension conditions and to limit the undesirable effect of the granules on the palate, it is necessary that, once the coating is completed, the final dimensions of the coated granules be comprised between 50 and 500 $\mu$m.

The so coated microgranules are mixed with suitable suspending, and aromatizing agents and additives and stored in this way, constituting formulations which can be suspended in aqueous vehicles.

Alternatively, the coated microgranules may be dispersed and kept in a dispersion in the suitable liquid vehicle containing the suspending, aromatizing, agents, and additives so as to obtain an immediately available dosage form.

Both formulations have the property of remaining stable at room temperature for long periods which can be measured in several months.

It is also an object of the present invention the possibility of obtaining with the above mentioned formulations beside the protracted effect, a prompt therapeutic response.

Such prompt response is obtained through the contemporaneous presence in the dosage formulation, beside the controlled release forms, also of a predermined quantity of the same active principle contained in the protracted release forms, a quantity which is mainly a function of the solubility of the active principle in the liquid selected as a vehicle. Such quantity of active principle may have been solubilized or introduced into the liquid form in an extemporaneous way.

A dose of active principle is thus immediately available which will bridge the time gap needed by the controlled release forms to exert their action.

The present invention is applicable to a great variety of drugs having various characteristics and, particularly in the form of extemporaneous suspensions, may be useful for such drugs which tend to be unstable when put in solution.

Particularly interesting results were obtained using theophylline as an active principle.

Theophylline has for a long time been considered the preferred drug for the treatment of acute and chronic bronchial constriction syndromes, and the therapy for its use provides for frequent admnistrations during the day and for long periods of time, up to the disappearance of the symptoms.

A pharmaceutical formulation allowing the controlled release of theophylline has therefore the double advantage of maintaining as much as possible the hematic levels in the area of therapeutic concentrations, and to limit the number of daily administrations, thus improving the compliance by the patient, and this in addition to the previously described advantages of the personally adapted dosage and of the practicality of use.

In detail, the present invention consists therefore of a controlled release therapeutic system dosable in liquid form comprising:

1) controlled release form of an active principle, particularly theophylline, having dimensions comprised between 50 and 500 $\mu$m, apt to easily remain in suspension in a liquid for a long time, consisting of:

1.1) an active principle suitably transformed by means of excipients into a microgranular nucleus having well defined technological and morphological characteristics, which are essential to assure the reproducibility and the uniform distribution of the successive film layers.

1.2) a first coating in contact with said microgranular nucleous for the purpose of forming a barrier insensitive to pH variations. Such barrier, operating as a diffusion membrane, allows a regulation of the release of the drug contained in the nucleus and, at the same time, thanks to its intrinsic characteristics, makes it possible to maintain the preestablished dimension limits.

1.3) a series of successive coatings overlaying the first, which, while keeping the dimensions of the granules in the predetermined limits, constitute an alternation of layers of a hydrophilic and lipophilic character, which can be regulated in number and succession so as to be adapted to the pharmacological characteristics of the used active principle. The therapeutic response is thus optimized and, at the same time, the complete dispersibility of the coated microgranules, at the moment of their suspension in the liquid vehicle, is obtained.

2) A vehicle system for the mentioned controlled release form alternatively consisting of:

2.1) a dry mixture of suspending agents, sweetening agents and of the controlled release forms described in 1) such as to obtain a formulation which can be reconstituted at the moment of usage. Such formulation has rehological characteristics apt to mantain in suspension the coated microgranules after their introduction in the aqueous solvent, and to keep the release characteristics unchanged for long periods of time.

2.2) a water solution of the above mentioned suspension-and sweetening agents in which the controlled release forms described under 1 may be suspended and kept in otpimal release conditions for long periods of time.

3) a programmed quantity of the active principle added to the 2.1) mixture or dissolved into the 2.2) solution, for the purpose of supplying an immediately effective therapeutic dose at the moment of administration.

According to the present invention we have thus found that, by wet kneading active principle with excipients, it is possible, according to known techniques, to obtain granules having an high content of active principle and having, in the final coating state, dimensions within 50 to 500 $\mu$m (preferably 125-300 $\mu$m), a uniform, almost spherical surface, an apparent density comprised between 300-800 g/l (preferably 500-600 g/l) and a very low friability.

The present method may be applied to active principles differing both for their physico-chemical characteristics and for the therapeutic class to which they belong.

The system is usable for the administration of a large variety of active principles, of acidic, basic or neutral character.

As an example, the system may be applied to anti-inflammatory, anti-histaminic, diuretic, gastrochinetic, anti-asthmatic drugs.

In particular the system is suitable for the administration of theophylline.

The employed excipients may be selected among those commonly employed in wet kneading, such as for example, dibasic calcium phosphate, lactose, microcrystalline cellulose, starch, talc, sugars, polyvinylpyrrolidone, polyvinylpyrrolidone-vinyl acetate copolymer, sodium phosphate, hydrogenated castor-oil, citric acid, tartaric acid.

The kneading liquid may be water or a solvent miscible with water, as for example ethyl alcohol and other commonly used alcohols, or a water-alcohol mixture.

Always according to the invention the granulate is then coated, in successive phases and according to known coating techniques, with films of different compositions.

As an example we list hereinbelow the materials which may form the three different types of films employed.

First coating on the granule

Mixture in various ratios of a first component, consisting of cellulose derivatives such as hydroxy-propylcellulose phtalate, ethylcellulose, carboxy-methyl-cellulose acetate, carboxy-methyl-cellulose acetate butyrate, or copolymers of esters of methacrylic and acrylic acid, methyl methacrylates, with a second component consisting of hydrogenated or partially hydrogenated cotton-, soy-, arachid-, castor - oil, with the addition of a suitable plasticizing agent such as diethyl phtalate, mono glyceryl acetate, polyethylenglycol, alkyl citrates.

Successive coatings:

Lipophilic component layer:
fatty substances such as: mono-, di-, triglycerids of fatty acids having from 6 to 32 carbon atoms, carnauba wax, beewax, candelilla wax, alcohols, fatty acids.

Hydrophilic component layer:
substances having either gastroresistant characteristics or not such as: cellulose acetophtalate, hydroxypropylmethylcellulose phtalate, hydroxypropylcellulose, hydroxypropylmethyl cellulose, copolymers of methacrylic-, acrylic- and methylmethacrylic acid esters,.

For this type of filming material, the contemporaneous presence of plasticizers may be useful.

As plasticizer one can employ, for instance, dietylphtalate, dybutylsebacate, triacetin, trialkyl citrates, acetylated vegetable oils and glycerids, polyethylene glycols, propylenglycol and others.

The selection of the most suitable plasticizer depends on whether it is employed when operating in a water medium or with the aid of organic solvents or mixtures of them.

As film solvents, in fact, one can use beside water, chlorinated solvents such as chloroform and others, and alcohols such as ethanol, methanol, isopropyl alcohol and others, as well as ketones such as acetone, methyl-ethyl ketone and others.

The film coating consists of a first layer deposited on the granulate, followed by a succession of one or more superimposed layers "onion-wise", consisting of lipophilic and hydrophilic suitably alternated materials. Except for the need of alternating lipophilic and hydrophilic layers, the sequence and the number of layers are basically determined by the characteristics of the active principle to be administered and by the desired release characteristics.

As examples we list herein below some combinations of possible coating sequences:

a) ethylcellulose and hydrogenated castor-oil followed by cellulose acetophtalate and plasticizer,

b) ethylcellulose and hydrogenated castor-oil followed by cellulose acetophtalate and plasticizer, then alternating layers of waxes and cellulose acetophtalate, concluding with a cellulose acetophtalate and plasticizer layer as external layer.

c) ethylcellulose and hydrogenated castor-oil followed by a layer consisting of a mixture of: gliceryl monostearate, beewax, cetyl and stearyl alcohol, followed by a layer of cellulose acetophtalate and plasticizer. If necessary the alternating sequence of lipophilic and hidrophilic layers may be repeated several time.

d) ethylcellulose and hydrogenated castor-oil followed by a layer consisting of a mixture of gliceryl monostearate, beewax, cetyl- and stearyl alcohol, alternating with layers of cellulose acetolphtalate and plasticizer.

e) coating sequences similar to the ones described under points a), b) and c) in which hydrogenated castor oil is substituted by diethylacetolphtalate or other plasticizers.

In agreement with the present invention, the granulate coated according to the methods described may be then introduced into a vehicle in the following forms:

a) suspension ready for use,

b) solid mixture which can be suspended extemporaneously at the moment of usage.

In addition to the amount of coated microgranules containing the dose of controlled release active principle, the elements constituting the vehicle for form a) are:

1 - a dose of active principle in solution, forming a readily absorbable fraction.

2 - suspending- and structural-agents such as cellulose esters, microcrystalline cellulose, alginic acid derivatives, polivinyl pyrrolidone derivatives.

3 - sugars, such as sucrose and sorbitol.

4 - buffers such as citric acid and sodium citrate, glycin and hydrocloric acid, sodium and potassium phosphates.

5 - preservatives and bacteriostatic agents such as p-hydroxybenzoic acid esters.

6 - aromatizing and sweetening agents such as saccharine and others.

7 - water or mixtures of water and solvents such as glycols, alcohols, glycerin.

Form b) on the other hand consists of a mixture of the coated granulate containing the active principle in a controlled release dose and a mixture consisting of a fraction of the prompt release drug together with the excipients described in points 2 to 6, and which may be granulated according to conventional methods.

In particular, the purpose of the pharmaceutical form in the extemporaneous suspension b), is to make it possible to use a vehicle also for those active principles which, in a aqueous vehicle, might present problems of chemical stability in the times foreseen for the therapeutic use.

We will now give methods, tables and examples only for the purpose of better illustrating the invention, showing its advantages and applicability, without, however, constituting a limitation of same. Even if in such examples reference is made to some known drugs only, it is evident that the present invention may be applied to a large number of active principles both of a similar or different structure.

EXAMPLE 1

A. Preparation of the microgranulate

The active principle and the excipient are introduced in a kneading vessel, the powders are mixed for a time sufficient to obtain a homogeneous mixture and then the kneading liquid is added.

The liquid is distributed in the homogeneous powder mix by spraying through a nozzle of a diameter which may vary between 0.5 and 2 mm, depending both on the spraying pressure which may vary between 0.5 and 6 Bar, and the type of wetting solution employed.

By varying the dimension of the nozzle one can obtain a more or less subdivided nebulization, so as to adjust the liquid distribution during the wetting step according to requirements.

The wetting liquid may be brought to the diffusion nozzle by means of a perystaltic pump, or a similar system, with a flux which may vary between 20 and 80 ml/min, depending on the liquid selected, and at any rate in such a way as to obtain a uniform distribution of the liquid in the powder mixture.

At the end of the wetting, the granulate is made into spheres mantaining the mixing of the mass for a time between 5 and 20 minutes, depending on the operative conditions and on the materials employed in the preceding steps. Once the formation of the spheres is terminated, the product is dried in a static oven or on a fluid bed according to known techniques, thus obtaining a granulate ready for film coating having a high content of active principle, a size between 50 and 500 $\mu$m and a spheroidal form which does not present sharp or discontinuous surfaces.

The powder fraction, below 50 $\mu$m, and the coarse fraction, above 500 $\mu$m, are separated and pulverized with the aid of a mill, and used again in successive operations. We report hereinbelow some compositions for 100 g granulate obtained with the described technique:

| Components | Weigth (g) |
|---|---|
| a) | |
| Theophylline | 80 |
| Bibasic bihydrated calcium phosphate | 10 |
| Talc | 5 |
| Polivinylpyrrolidone vinyl acetate | 5 |
| b) | |
| Hydrated, micronized theophylline | 24 |
| Microcrystalline cellulose | 15 |
| Corn starch | 15 |
| Lactose | 39 |
| Polivinylpyrrolidone | 7 |
| c) | |
| Micronized Ketorolac | 2 |
| Microcrystalline cellulose | 15 |
| Corn starch | 15 |
| Lactose | 58 |
| Polivinylpirrolidone | 10 |
| d) | |
| Micronized hydrated theofilline | 80 |
| Hydrogenated castor-oil | 10 |
| Talc | 5 |
| Polivinylpyrrolidone vinyl acetate | 5 |
| e) | |
| Anhydrous micronized theofilline | 80 |

| Monohydrated citric acid | 9.6 |
| Bihydrated, bibasic sodium phosphate | 0.4 |
| Talc | 2.5 |
| Polivinylpirrolidone vinyl acetate | 7.5 |

B. Film coating of the microgranulate

For applying the film coating one can employ known techniques, such as conventional pans, fluid bed systems and other similar methods.

The coating material may be applied by dissolving the film forming materials into organic solvents or using aqueous dispersions of the materials, or even by applying directly the materials in the molten state.

We report hereinbelow some examples of the coating layer composition for 100 ml of film forming solution.

a) first film forming coating in contact with the granule

| Components | |
|---|---|
| Ethylcellulose | 1 g |
| Hydrogenated castor-oil | 4 g |
| Chloroform | 65 ml |
| Ethanol | 35 ml |
| or: | |
| Ethylcellulose | 4 g |
| Diethylphtalate | 1 g |
| Chloroform | 65 ml |
| 95% ethanol | 35 ml |
| or: | |
| Ethylcellulose | 4 g |
| Hydrogenated Castor-oil | 1 g |
| Chloroform | 65 ml |
| 95% ethanol | 35 ml |

b) Hydrophilic film coating

| Components | |
|---|---|
| Cellulose acetophtalate | 5 g |
| Dietylphtalate | 1.25 g |
| Acetone | 75 ml |
| Isopropanol | 25 ml |

c) Lipophilic film coating

8

| Components | |
|---|---|
| Gliceryl monostearate | 9 g |
| White beewax | 0.8 g |
| Cetyl alcohol | 0.1 g |
| Stearyl alcohol | 0.1 g |
| Chloroform | 90 ml |
| Methanol | 10 ml |

Film forming method

The granulate spheres are introduced in a Glatt fluid bed apparatus provided with a 1.2 mm nozzle and the apparatus is preheated to 35°C. The a) coating solution is sprayed feeding the nozzle with a peristaltic pump, keeping a constant flux and nebulizing by means of air injected at a pressure of 2 Bar. Once the coating with solution a) is terminated, coating of successive alternating layers using solutions b) and c) is performed in a similar way. If required by the desired release- and active principle characteristics, the filming with materials of solutions b) and c) may be repeated several times. In general the last coating layer consists of solution b). When the filming of the last layer is completed, the coated granulate is dried, for example keeping it into the Glatt at 40°C for at least 30', after which the coated product is ready to be dispersed in the liquid vehicle.

C. Preparation of a ready liquid formulation

In a portion of the water necessary for the preparation, the preservatives are dissolved and the structure forming materials dispersed until swollen, then the active principle constituting the ready dose and the excipients and sweetening agents required are added. After adding the buffer and the release controlled granulate containing the active principle, the aromatizing substance is added and the whole is brought to the desired volume.

We describe hereinbelow an example of a composition for preparing 100 ml of a dosage liquid corresponding to 3 g anhydrous theophylline.

Components                                          Dose

Theophylline (dose ready to use)          0.6 g

Coated granulate (retard dose)

| | |
|---|---|
| corresponding to  anhydrous theophylline | 2.4 g |
| Microgranular cellulose | 2.0 g |
| Sodium carboxymethylcellulose | 0.26 g |
| Hydroxyetylcellulose | 0.05 g |
| Sucrose | 14.00 g |
| Sodium saccharinate | 0.10 g |
| Methyl p-hydroxybenzoate | 0.15 g |
| Propyl p-hydroxybenzoate | 0.05 g |
| Glycocol | 0.73 g |
| 1N hydrochloric acid | 1.2  ml |
| Mint-artichoke aroma | 0.5  g |
| Water | q.s. to 100 ml |

EXAMPLE 2

Preparation of a liquid formulation to be reconstituted

We describe an example of a composition for 100 ml of extemporaneous suspension:

| Components | Dose (g) |
|---|---|
| Theophylline (ready to use) | 0.60 |
| Coated granulate (retard dose) | |
| corresponding to anhydrous Theophylline | 2.40 |
| Sodium alginate | 1.00 |
| Sodium citrate | 0.22 |
| Citric Acid | 0.41 |
| Sodium saccharinate | 0.20 |
| Sodium methyl p-hydroxybenzoate | 0.15 |

| Sodium propyl p-hydroxybenzoate | 0.05 |
|---|---|
| Orange aroma powder | 0.10 |
| Sucrose | 14.00 |

The retard granulate prepared as described in example 1 and coated with successive film layers according to the method described at point B., is mixed in a suitable ratio with a granulate constituting the ready release portion. The latter is prepared by kneading in water a mixture consisting of theophylline, sucrose, sodium alginate, sodium saccharinate, sodium citrate, citric acid, sodium methyl- and propyl-p-hydroxybenzoate. After granulating on a 0.300 mm mesh sieve, the product is dried up to a constant humidity content and then is admixed with the powdered aroma.

Release control

The release control is performed using powder apparatus II (paddle) of the United States Pharmacopea XXI Ed., operating at 50 r/min using 900 ml of a phosphate buffer at pH 7.4 having the following composition:

| Bihydrated bibasic sodium phosphate | 14.40 g |
|---|---|
| Bihydrated monobasic sodium phosphate | 2.96 g |
| Demineralized water | q.s.to    1000 ml |

The determination of the released active principle is performed spectrophotometrically or through HPLC according to known methods.

The release tests are carried out on microgranules with film coatings which differ in type and composition, and employed in such quantity so as not to reach the saturation limits of the dissolution medium for the various tested active principles (sink condition).

Various types of film coating are prepared for the dissolution tests in vitro.

The following dissolution tables will evidence the advantages of the invention with respect to possible coating alternatives.

TABLE 1

| Dissolution of granules having dimensions of 125-250 mcm with multilayer coating and differing one from the other only because of the thickness of the first coating layer. | | | | | |
|---|---|---|---|---|---|
| Time (hrs) | A | B | C | D | E |
| | (dissolution %) | | | | |
| 1 | 95 | 1,7 | 8,3 | 8,3 | 10,8 |
| 2 | 100 | 2,5 | 10,8 | 12,5 | 17,9 |
| 4 | | 4,2 | 14,2 | 20,0 | 29,3 |
| 6 | | 5,8 | 16,7 | 25,0 | 34,7 |
| 8 | | 8,3 | 19,2 | 29,2 | 40,4 |

Thickness of the first coating layer = A: not coated, B: 10-13 mcm C: 9-10 mcm, D: 6-8 mcm, E: 5-6 mcm.

The higher the thickness of the first layer, the lower the drug release.

TABLE 2

Dissolution comparison between granules with two layer coating and with multi-layer coating of the same materials.

In these cases the microgranules were not coated with the first ethylcellulose-hydrogenated castor oil layer.

(F): coating with a wax layer and with a layer of cellulose acetophtalate (CAP).

(G): coating with several wax and CAP alternating layers.

| Time (hrs) | F (dissolution %) | G |
|---|---|---|
| 1 | 4,2 | 2,3 |
| 2 | 16,6 | 7,4 |
| 4 | 58,3 | 33,3 |
| 6 | 96,0 | 63,0 |
| 8 | 100 | 84,0 |

Because of the reduced dimensions of the particles to be coated, in case (F) in order to obtain release values comparable to case (G), one must distribute a high amount of waxes (50-60 % of the total granule weight) going thus beyond the predetermined dimension limits for the coated microgranule.

TABLE 3

Comparison of the dissolution between a complete multilayer coating (E) and a multilayer coating containing only waxes and CAP (G) and no first ethylcellulose-hydrogenated castor oil layer.

| Time (hrs) | E | G |
|---|---|---|
| | (dissolution %) | |
| 1 | 10,8 | 2,3 |
| 2 | 17,9 | 7,4 |
| 4 | 29,3 | 33,3 |
| 6 | 34,7 | 63,0 |
| 8 | 40,4 | 84,0 |

The advantage of the complete multiple coating (E) consists in keeping reduced granule dimensions while mantaining the efficacy in delaying the release, notwithstanding the high surface area of the particles.

TABLE 4

Comparison of the dissolution of multilayer coatings differing only because of the number of layers after the first.

Description of the layers:

a: ethylcellulose -hydrogenated castor oil mixture

b: CAP

c: waxes

Formulation and coatings:

(H) = a/b, (I) = a/b/c/b, (L)=a/b/c/b/c/b.

| Time (hrs) | H | I | L |
|---|---|---|---|
| | (dissolution %) | | |
| 1 | 10,0 | 14,0 | 8,0 |
| 2 | 12,0 | 17,0 | 12,0 |
| 4 | 14,0 | 23,0 | 17,0 |
| 6 | 18,0 | 27,5 | 22,0 |
| 8 | 21,0 | 31,0 | 27,0 |

It is evident how by alternating hydrophilic and lypophilic layers one can advantageously change the rate of dissolution, making it also more constant with time.

TABLE 5

| Time (hrs) | Dissolution of multilayer coating microgranules containing Naproxene (M), Ketorolac Trometamina (O) and Theofilline (Q) in comparison with the dissolution of non coated granules containing respectively the same active principles (N, P, R,). | | | | | |
|---|---|---|---|---|---|---|
| | M | N | O | P | Q | R |
| | (dissolution %) | | | | | |
| 1 | 26,0 | 85,0 | 29,0 | 95,0 | 26,5 | 96,0 |
| 2 | 36,0 | 95,0 | 36,5 | 100 | 31,2 | 100 |
| 4 | 48,0 | 100 | 49,0 | | 45,7 | |
| 6 | 57,0 | | 60,0 | | 54,8 | |
| 8 | 65,0 | | 72,0 | | 61,9 | |

The table shows the possibility of employing the multilayer film forming for substances with different physico-chemical characteristics.

Release tests were also carried out on film covered microgranules suspended in a suitable aromatized vehicle containing 20 % theofilline as ready dose, in an amount such as not to reach the saturation limits of the dissolution medium (sink condition).

For the dissolution tests in vitro various formulation types were prepared taking mainly into account two parameters: the composition of the microgranulates and the type of coating. In TABLE 6 some examples are reported. In all of them the first film coating, which regulates the release of the active principle, is followed by five alternating hydrophilic and lipophilic coating layers.

## TABLE 6

Dissolution of suspensions containing granules having dimensions between 125 and 250 μm with multilayer coating, and differing one from the other because of the characteristics of the microgranulate or the ones of the first coating layer

| Time (hrs) | A | S | T | U | V |
|---|---|---|---|---|---|
| | | (dissolution %) | | | |
| 1 | 95,00 | 35,59 | 34,41 | 39,49 | 32,01 |
| 2 | 100 | 42,64 | 42,33 | 46,75 | 49,50 |
| 4 | | 53,29 | 52,77 | 53,98 | 59,89 |
| 6 | | 61,05 | 60,68 | 58,83 | 67,41 |
| 8 | | 66,68 | 65,85 | 62,33 | 72,70 |

A = Microgranulate without coating

S = Microgranulate consisting of a mixture of theophylline, calcium phosphate, talc, polyvinylpyrrolidone vinyl acetate, coated with a first layer consisting of ethyl cellulose and dietylphtalate and with a series of 5 alternating lipophilic and hydrophilic coatings.

T = Microgranulate consisting of a mixture of theophylline, hydrogenated castor oil, talc, polivynilpyrrolidone vinylacetate, coated with a first layer consisting of ethyl cellulose and dietylphtalate and with a serie of 5 alternating lipophilic and hydrophilic layers.

U = Microgranulate consisting of a mixture of theophylline, calcium phosphate, talc, polivinylpyrrolidone vinyl acetate, coated with a first layer consisting of ethyl cellulose and hydrogenated castor oil and with a series of five alternating lipophilic and hydrophylic layers.

V = Microgranulate consisting of a mixture of theophylline, citric acid, sodium phosphate, talc, polyvinylpyrrolidone vinylacetate, coated with a first layer consisting of ethyl cellulose and diethylphtalate, and with a series of 5 alternating lipophilic and hydrophilic layers.

From the data of TABLE 6 it is evident how the coating of the granules can allow to adjust the release of an active principle.

Stability

The stability controls were performed by evaluating the behaviour with time of the formulations prepared according to the description in example 1 (liquid formulation ready to use ) and in example 2 (formulation to be reconstituted in liquid form).

The stability with time of the release characteristics of the drug was verified by dissolution tests performed according to what previously described in the paragraph relating to the release control.

TABLE 7

| Stability at room temperature of liquid formulations ready to use (W: initial) (X: after 6 months) and reconstituted (Y: initial, Z: after 6 months), both containing theophylline as active principle. | | | | |
|---|---|---|---|---|
| Time (hrs) | W | X | Y | Z |
| | (dissolution %) | | | |
| 0 | 19,4 | 25,6 | 21,0 | 21,5 |
| 1 | 26,5 | 36,1 | 29,5 | 30,0 |
| 2 | 31,2 | 41,7 | 30,0 | 34,2 |
| 4 | 45,7 | 54,5 | 31,0 | 40,8 |
| 6 | 54,9 | 62,2 | 35,5 | 47,5 |
| 8 | 58,7 | 66,2 | 45,1 | 48,5 |

Relative bioavailability control

The relative bioavailability of the theophylline formulations bioavailability described in table 6 was verified by a pharmacokinetic study performed on Beagle dogs. Dogs of both sexes, of weight comprised between 12 and 14 Kg, were treated in two crossover design studies with a single oral dose of liquid theophylline formulations containing each 300 mg of active principle. A controlled release theophylline formulation in tablets (THEO-DUR[R] 300) was used as reference standard. Blood specimens were taken at 0, 0.5, 1.0, 1.5, 2, 3, 4, 6, 8, 11, 24, 28, 32 hours after administration and were then analysed to determine, by HPLC method, the plasmatic theophylline concentrations. The bioavailability parameters are summarized in TABLES 8,9. For the formulations and the standard are reported the mean values relative to: area under the plasma concentration/time curves between 0 and 32 hours ($AUC_{0-32}$), time for reaching the highest concentration (Tmax), and the highest concentration reached (Cmax), S.E. is the standard error and CV represents the variation coefficient on the average.

TABLE 8

Bioavailability parameters of two controlled release theophylline formulations described in table 6, in comparison with a THEO-DUR[R] 300 formulation.

| | THEO-DUR[R] | S | T |
|---|---|---|---|
| | $AUC_{0-32}$ (h.mcg/ml) | | |
| average | 216,01 | 184,49 | 223,71 |
| ±S.E. | 3,36 | 3,60 | 9,63 |

| | | | |
|---|---|---|---|
| CV (%) | 2,70 | 3,38 | 7,45 |

$T_{max}$ (hours)

| | | | |
|---|---|---|---|
| average | 4,33 | 4,67 | 4,33 |
| ±S.E. | 0,72 | 0,54 | 0,72 |
| CV (%) | 28,78 | 20,20 | 28,78 |

Cmax (mcg/ml)

| | | | |
|---|---|---|---|
| average | 20,18 | 15,01 | 20,38 |
| ± S.E. | 2,29 | 0,71 | 2,75 |
| CV (%) | 19,64 | 8,24 | 23,36 |

## TABLE 9

Bioavailability parameters of two controlled release theophylline formulations described in table 6, in comparison with a THEO-DUR[r] 300 formulation.

| | THEO-DUR[r] | U | V |
|---|---|---|---|

$AUC_{0-32}$ (h.mcg/ml)

| | | | |
|---|---|---|---|
| average | 286,74 | 242,67 | 252,17 |
| ± S.E. | 3,80 | 21,45 | 8,40 |
| CV (%) | 2,30 | 15,31 | 5,77 |

Tmax (hours)

| | | | |
|---|---|---|---|
| average | 6,67 | 5,67 | 5,33 |
| ± S.E. | 1,91 | 1,19 | 1,44 |
| CV (%) | 44,50 | 36,26 | 46,77 |

Cmax (mcg/ml)

| | | | |
|---|---|---|---|
| average | 17,72 | 16,79 | 17,63 |
| ± S.E. | 1,55 | 0,22 | 1,29 |
| CV (%) | 15,16 | 2,22 | 12,68 |

18

The data in the tables show that it is possible, by a suitable variation in the composition of the formulations, to obtain a good bioavailability of the active principle, which is at any rate comparable to the one of a solid formulation of the same active principle having well-known therapeutic characteristics.

**Claims**

**Claims for the following Contracting States : AT, BE, CH, DE, DK, FR, GB, IT, LI, LU, NL, SE**

1. Controlled release therapeutic system for formulations relating to liquid pharmaceutical forms consisting of:

   a) microgranules containing the active principle and suitable excipients, having dimensions which easily allow their suspension in a liquid medium after having been conveniently coated with polymeric materials,

   b) a series of multilayer polymeric lipidic and wax-like coatings covering said microgranules while mantaining their dimension limits, so as to allow the controlled release of the active principle contained in the microgranules in times which can be predetermined, and at the same time such as to assure the complete dispersion of the coated microgranules in the liquid vehicle and furthermore the conservation of the release characteristics for a long time,

   c) a liquid administration vehicle for said coated microgranules, characterized in that it contains, in addition to suitable additives, an immediately available amount of the same active principle contained in the controlled release microgranules.

2. Therapeutic system according to claim 1, characterized in that the active principle used is theophylline and/or its pharmaceutical acceptable salts.

3. Therapeutic system according to claim 1, characterized in that the microgranules in a) have a high content of active principle, uniform surface, almost spherical shape, an apparent density comprised between 300 and 800 g/l and a low friability.

4. Therapeutic system according to claim 1, characterized in that the series of coatings mentioned in b) consists of a first layer on the surface of the microgranules described in a) having the purpose of forming a barrier non-sensitive to the pH variations, which will permit a control of the active principle release within the limits of the predetermined dimensions, and of successive layers coated in sequence one upon the other, which constitute an alternation of hydrophilic and lipophilic layers in such a way that, as a function of the nature and of the therapeutic requirements of the active principle, they further regulate the active principle release ensuring at the same time the dispersibility of the coated granules in the liquid vehicle.

5. Therapeutic system according to claim 4, characterized in that the first layer of material deposited on the microgranule nucleus consists of a mixture, in variable proportions, of a first component selected among the derivatives of cellulose or of acrylic and metacrylic acid with a second component consisting of hydrogenated or partially hydrogenated vegetable oils and of a suitable plasticizing materials.

6. Therapeutic system according to claim 5, characterized in that the mixture of the two components is in a ratio reciprocally variable between 20 and 80 %.

7. Therapeutic system according to claim 4, characterized in that the materials constituting the layer or layers having hydrophilic nature are selected alternatively among the polymers derived from cellulose and from acrylic and metacrylic acid.

8. Therapeutic system according to claim 7, characterized in that the materials constituting the layer or the layers having hydrophilic nature are utilized in the presence of plasticizers.

9. Therapeutic system according to claim 4, characterized in that the materials constituting the layer or the layers having lipophilic character are selected among lipids such as fatty acid mono-, di-, three-glycerids, waxes, fatty alcohols, fatty acids.

**10.** Therapeutic system according to claims 1, 4, and 7 to 9, characterized in that the layers having a lipophilic and hydrophilic nature are distributed in alternance, the sequence and number depending on the diffusional and physico-chemical characteristics of the selected active principle.

**11.** Therapeutic system according to claims 1 to 10, characterized in that the coated microgranules have dimensions comprised between 50 and 500 um.

**12.** Therapeutic system according to claims 1 to 10, characterized in that the coated microgranules have dimensions comprised between 125 and 250 $\mu$m.

**13.** Therapeutic system according to claim 1, characterized in that vehicle c) contains in addition to the active principle as immediately available dose: suspending-, structurating-, sweetening-, buffering-, preserving-, and aromatizing substances.

**14.** Therapeutic system characterized in that the mixture consisting of the components of the system as claimed in anyone of claims 1 to 12 is suspended in water or in a mixture of water and water miscible solvents, to obtain a liquid dosage immediately usable formulation.

**15.** Therapeutic system according to claim 14, characterized in that the suspension in the liquid vehicle maintains constant, for a period of various months, the release characteristics of the coated microgranules.

**16.** Therapeutic system according to claims 1 to 13, characterized in that the mixture of the system components may constitute an extemporaneous formulation which can be suspendend in an aqueous solvent at the moment of the therapeutic utilization.

**17.** Therapeutic system according to claim 16, characterized in that the formulation constituted by a solid mixture which can be suspended extemporaneously may be utilized in particular for active principles of low stability in a water medium.

**18.** Therapeutic system according to claims 1 to 17, characterized in that it is applicable, after previous adjustment of the components, for the administration and dosage of active principles having an acidic, basic or neutral character.

**Claims for the following Contracting States : ES, GR**

**1.** Process for the preparation of controlled release therapeutic system for formulations relating to liquid pharmaceutical forms consisting of:
a) microgranules containing the active principle and suitable excipients, having dimensions which easily allow their suspension in a liquid medium after having been conveniently coated with polymeric materials,
b) a series of multilayer polymeric lipidic and wax-like coatings covering said microgranules while mantaining their dimension limits, so as to allow the controlled release of the active principle contained in the microgranules in times which can be predetermined, and at the same time such as to assure the complete dispersion of the coated microgranules in the liquid vehicle and furthermore the conservation of the release characteristics for a long time,
c) a liquid administration vehicle for said coated microgranules, characterized in that it contains, in addition to suitable additives, an immediately available amount of the same active principle contained in the controlled release microgranules, comprising the following steps:
i) said active principle is first granulated by wet kneading with said excipients;
ii) said microgranules are coated with the above mentioned series of coatings;
iii) the coated granulate is introduced into said vehicle;
iv) said immediately available amount of active principle is solubilized or introduced in the liquid form in an extemporaneous way.

**2.** Process according to claim 1, characterized in that the active principle used is theophylline and/or its pharmaceutical acceptable salts.

3. Process according to claim 1, characterized in that the microgranules in a) have a high content of active principle, uniform surface, almost spherical shape, an apparent density comprised between 300 and 800 g/l and a low friability.

4. Process according to claim 1, characterized in that the series of coatings mentioned in b) consists of a first layer on the surface of the microgranules described in a) having the purpose of forming a barrier non-sensitive to the pH variations, which will permit a control of the active principle release within the limits of the predetermined dimensions, and of successive layers coated in sequence one upon the other, which constitute an alternation of hydrophilic and lipophilic layers in such a way that, as a function of the nature and of the therapeutic requirements of the active principle, they further regulate the active principle release ensuring at the same time the dispersibility of the coated granules in the liquid vehicle.

5. Process according to claim 4, characterized in that the first layer of material deposited on the microgranule nucleus consists of a mixture, in variable proportions, of a first component selected among the derivatives of cellulose or of acrylic and metacrylic acid with a second component consisting of hydrogenated or partially hydrogenated vegetable oils and of a suitable plasticizing materials.

6. Process according to claim 5, characterized in that the mixture of the two components is in a ratio reciprocally variable between 20 and 80 %.

7. Process according to claim 4, characterized in that the materials constituting the layer or layers having hydrophilic nature are selected alternatively among the polymers derived from cellulose and from acrylic and metacrylic acid.

8. Process according to claim 7, characterized in that the materials constituting the layer or the layers having hydrophilic nature are utilized in the presence of plasticizers.

9. Process according to claim 4, characterized in that the materials constituting the layer or the layers having lipophilic character are selected among lipids such as fatty acid mono-, di-, three-glycerids, waxes, fatty alcohols, fatty acids.

10. Process according to claims 1, 4, and 7 to 9, characterized in that the layers having a lipophilic and hydrophilic nature are distributed in alternance, the sequence and number depending on the diffusional and physico-chemical characteristics of the selected active principle.

11. Process according to claims 1 to 10, characterized in that the coated microgranules have dimensions comprised between 50 and 500 $\mu$m

12. Process according to claims 1 to 10, characterized in that the coated microgranules have dimensions comprised between 125 and 250 $\mu$m.

13. Process according to claim 1, characterized in that vehicle c) contains in addition to the active principle as immediately available dose: suspending-, structurating-, sweetening-, buffering-, preserving-, and aromatizing substances.

14. Process characterized in that the mixture consisting of the components of the system as claimed in claims 1 to 12 is suspended in water or in a mixture of water and water miscible solvents, to obtain a liquid dosage immediately usable formulation.

15. Process according to claim 14, characterized in that the suspension in the liquid vehicle maintains constant, for a period of various months, the release characteristics of the coated microgranules.

16. Process according to claims 1 to 13, characterized in that the mixture of the system components may constitute an extemporaneous formulation which can be suspendend in an aqueous solvent at the moment of the therapeutic utilization.

EP 0 359 195 B1

**17.** Process according to claim 16, characterized in that the formulation constituted by a solid mixture which can be suspended extemporaneously may be utilized in particular for active principles of low stability in a water medium.

**18.** Process according to claims 1 to 17, characterized in that it is applicable, after previous adjustment of the components, for the administration and dosage of active principles having an acidic, basic or neutral character.

**Patentansprüche**

**Patentansprüche für folgende Vertragsstaaten : AT, BE, CH, DE, DK, FR, GB, IT, LI, LU, NL, SE**

**1.** Therapeutisches System mit kontrollierter Wirkstoffabgabe für Zubereitungen, welche flüssige pharmazeutische Formen betreffen, bestehend aus
a) Mikrogranulaten, welche den Wirkstoff und geeignete Excipienten enthalten und eine solche Größe aufweisen, welche deren Suspension in einem flüssigen Medium erlauben, nachdem diese in vorteilhafter Weise mit einem polymeren Material überzogen wurden,
b) einer Folge von vielschichtigen polymeren lipidischen und wachsähnlichen Überzügen, welche die genannten Mikrogranulate unter Erhaltung ihrer Größe bedecken, so daß diese die kontrollierte Wirkstoffabgabe des in den Mikrogranulaten enthaltenen Wirkstoffes in vorherbestimmbarer Zeit erlauben und gleichzeitig die vollständige Dispersion der überzogenen Mikrogranulate in einem flüssigen Medium sicherstellen und weiterhin den Erhalt der Freisetzungseigenschaften über einen langen Zeitraum bewirken,
c) einem flüssigen Träger zur Verabreichung der genannten überzogenen Mikrogranulate, dadurch gekennzeichnet, daß dieser zusätzlich zu geeigneten Zusatzstoffen eine sofort verfügbare Menge des selben Wirkstoffes enthält, welcher auch in den kontrolliert freisetzenden Mikrogranulaten enthalten ist.

**2.** Therapeutisches System nach Anspruch 1, dadurch gekennzeichnet, daß der verwendete Wirkstoff Theophyllin und/oder eines seiner pharmazeutisch annehmbaren Salze ist.

**3.** Therapeutisches System nach Anspruch 1, dadurch gekennzeichnet, daß die Mikrogranulate in a) einen hohen Gehalt an Wirkstoff, gleichförmige Oberflächen, nahezu kugelige Form, eine scheinbare Dichte zwischen 300 und 800 g/l und eine geringe Friabilität aufweisen.

**4.** Therapeutisches System nach Anspruch 1, dadurch gekennzeichnet, daß die Folge von in b) genannten Überzügen aus einer ersten Schicht auf der Oberfläche der in a) beschriebenen Mikrogranulate besteht, welche die Aufgabe hat eine Sperre zu bilden, die gegenüber den pH-Wert-Änderungen unempfindlich ist, und welche die Kontrolle der Wirkstoffabgabe innerhalb der vorherbestimmten Grenzen erlaubt, und aus nachfolgenden Schichten, welche in einer Folge nacheinander aufgebracht werden, daß sie einen Wechsel von hydrophilen und lipophilen Schichten in einer solchen Weise darstellen, daß sie als gesetzmäßige Wirkung und entsprechend den therapeutischen Anforderungen des Wirkstoffes die Freisetzung des Wirkstoffes regulieren und gleichzeitig die Dispersibilität der beschichteten Granulate in dem flüssigen Medium sicherstellen.

**5.** Therapeutisches System nach Anspruch 4, dadurch gekennzeichnet, daß die erste Schicht des auf den Mikrogranulatkern aufgetragenen Materials aus einer Mischung besteht, welche sich in wechselnden Mengenverhältnissen aus einer ersten Komponente, welche aus Derivaten der Cellulose oder der Acryl- und Metacrylsäure ausgewählt ist, und einer zweiten Komponente zusammensetzt, welche aus hydrierten oder teilweise hydrierten pflanzlichen Ölen und geeigneten Plastifizierungsstoffen besteht.

**6.** Therapeutisches System nach Anspruch 5, dadurch gekennzeichnet, daß die Mischung der beiden Bestandteile in einem variablen reziproken Verhältnis zwischen 20 und 80 % vorliegt.

**7.** Therapeutisches System nach Anspruch 4, dadurch gekennzeichnet, daß die Stoffe, welche die Schicht oder Schichten bilden, die hydrophile Eigenschaften haben, alternativ aus den aus Cellulose oder aus Acryl- und Metacrylsäure erhaltenen Polymeren ausgewählt sind.

22

**8.** Therapeutisches System nach Anspruch 7, dadurch gekennzeichnet, daß die Stoffe, welche die Schicht oder die Schichten mit hydrophilen Eigenschaften bilden, in Gegenwart von Plastifizierungsmitteln verwendet werden.

**9.** Therapeutisches System nach Anspruch 4, dadurch gekennzeichnet, daß die Stoffe, welche die Schicht oder die Schichten mit lipophilem Charakter bilden, aus Lipiden wie Fettsäuremono-, di- oder triglyceriden, Wachsen, Fettalkoholen und Fettsäuren ausgewählt sind.

**10.** Therapeutisches System nach den Ansprüchen 1, 4 und 7 bis 9, dadurch gekennzeichnet, daß die Schichten mit lipophilen und hydrophilen Eigenschaften in alternierender Folge verwendet werden, wobei deren Folge und Anzahl von den Diffusions- und physikochemischen Eigenschaften des ausgewählten Wirkstoffes abhängen.

**11.** Therapeutisches System nach den Ansprüchen 1 bis 10 dadurch gekennzeichnet, daß die beschichteten Mikrogranulate eine Größe zwischen 50 und 500 μm aufweisen.

**12.** Therapeutisches System nach den Ansprüchen 1 bis 10, dadurch gekennzeichnet, daß die beschichteten Mikrogranulate eine Größe zwischen 125 und 250 μm aufweisen.

**13.** Therapeutisches System nach Anspruch 1, dadurch gekennzeichnet, daß der Träger c) neben der sofort verfügbaren Dosis des Wirkstoffes suspendierende, strukturgebende, süßende, puffernde, konservierende und aromatisierende Substanzen enthält.

**14.** Therapeutisches System, dadurch gekennzeichnet, daß die Mischung aus den Komponenten des in einem der Ansprüche 1 bis 12 beanspruchten Systems in Wasser oder in einer Mischung aus Wasser und wassermischbaren Lösemitteln suspendiert wird, um eine flüssige Dosis in einer sofort verwendbaren Formulierung zu ergeben.

**15.** Therapeutisches System nach Anspruch 14, dadurch gekennzeichnet, daß die Suspension im flüssigen Träger über einen Zeitraum von mehreren Monaten die Freisetzungseigenschaften der beschichteten Mikrogranulate konstant hält.

**16.** Therapeutisches System nach den Ansprüchen 1 bis 13, dadurch gekennzeichnet, daß die Mischung der Systemkomponenten aus einer extemporanen Zubereitung besteht, welche dann in einem wäßrigen Lösemittel zum Zeitpunkt der therapeutischen Verwendung suspendiert werden kann.

**17.** Therapeutisches System nach Anspruch 16, dadurch gekennzeichnet, daß die durch eine feste Mischung, welche extemporan suspendiert werden kann, bewirkte Formulierung insbesondere für solche Wirkstoffe verwendet wird, welche in wäßrigem Medium eine geringe Stabilität aufweisen.

**18.** Therapeutisches System nach den Ansprüchen 1 bis 17, dadurch gekennzeichnet, daß dieses nach vorhergehender Einstellung der Bestandteile für die Verabreichung und Dosierung von Wirkstoffen geeignet ist, welche sauren, basischen oder neutralen Charakter haben.

**Patentansprüche für folgende Vertragsstaaten : ES, GR**

**1.** Verfahren zur Herstellung therapeutischer Systeme mit kontrollierter Wirkstoffabgabe für Zubereitungen, welche flüssige pharmazeutische Formen betreffen, bestehend aus
a) Mikrogranulaten, welche den Wirkstoff und geeignete Excipienten enthalten und eine solche Größe aufweisen, welche deren Suspension in einem flüssigen Medium erlauben, nachdem diese in vorteilhafter Weise mit einem polymeren Material überzogen wurden,
b) einer Folge von vielschichtigen polymeren lipidischen und wachsähnlichen Überzügen, welche die genannten Mikrogranulate unter Erhaltung ihrer Größe bedecken, so daß diese die kontrollierte Wirkstoffabgabe des in den Mikrogranulaten enthaltenen Wirkstoffes in vorherbestimmbarer Zeit erlauben und gleichzeitig die vollständige Dispersion der überzogenen Mikrogranulate in einem flüssigen Medium sicherstellen und weiterhin den Erhalt der Freisetzungseigenschaften über einen langen Zeitraum bewirken,

c) einem flüssigen Träger zur Verabreichung der genannten überzogenen Mikrogranulate, dadurch gekennzeichnet, daß dieser zusätzlich zu geeigneten Zusatzstoffen eine sofort verfügbare Menge des selben Wirkstoffes enthält, welcher auch in den kontrolliert freisetzenden Mikrogranulaten enthalten ist, und wobei das Verfahren die folgenden Schritte umfaßt:

   i) anfängliches Granulieren des genannten Wirkstoffes durch feuchtes Verkneten mit den genannten Excipienten;
   ii) Überziehen der genannten Mikrogranulate mit der oben erwähnten Folge von Überzügen;
   iii) Einführung der überzogenen Granulate in den genannten Träger;
   iv) Lösung der genannten sofort verfügbaren Menge des Wirkstoffes oder Einführung desselben in die flüssige Formulierung auf einem extemporanen Wege.

2. Verfahren nach Anspruch 1, dadurch gekennzeichnet, daß der verwendete Wirkstoff Theophyllin und/oder eines seiner pharmazeutisch annehmbaren Salze ist.

3. Verfahren nach Anspruch 1, dadurch gekennzeichnet, daß die Mikrogranulate in a) einen hohen Gehalt an Wirkstoff, gleichförmige Oberflächen, nahezu kugelige Form, eine scheinbare Dichte zwischen 300 und 800 g/l und eine geringe Friabilität aufweisen.

4. Verfahren nach Anspruch 1, dadurch gekennzeichnet, daß die Folge von der in b) genannten Überzüge aus einer ersten Schicht auf der Oberfläche der in a) beschriebenen Mikrogranulate besteht, welche die Aufgabe hat, eine Sperre zu bilden, die gegenüber den pH-Wert-Änderungen unempfindlich ist, und welche die Kontrolle der Wirkstoffabgabe innerhalb der vorherbestimmten Grenzen erlaubt, und aus nachfolgenden Schichten, welche in einer Folge nacheinander aufgebracht werden, daß sie einen Wechsel von hydrophilen und lipophilen Schichten in einer solchen Weise darstellen, daß sie als gesetzmäßige Wirkung und entsprechend den therapeutischen Anforderungen des Wirkstoffes die Freisetzung des Wirkstoffes regulieren und gleichzeitig die Dispersibilität der beschichteten Granulate in dem flüssigen Medium sicherstellen.

5. Verfahren nach Anspruch 4, dadurch gekennzeichnet, daß die erste Schicht des auf den Mikrogranulatkern aufgetragenen Materials aus einer Mischung besteht, welche sich in wechselnden Mengenverhältnissen aus einer ersten Komponente, welche aus Derivaten der Cellulose oder der Acryl- und Metacrylsäure ausgewählt ist und einer zweiten Komponente zusammensetzt, welche aus hydrierten oder teilweise hydrierten pflanzlichen Ölen und geeigneten Plastifizierungsstoffen besteht.

6. Verfahren nach Anspruch 5, dadurch gekennzeichnet, daß die Mischung der beiden Bestandteile in einem variablen reziproken Verhältnis zwischen 20 und 80 % vorliegt.

7. Verfahren nach Anspruch 4, dadurch gekennzeichnet, daß die Stoffe, welche die Schicht oder Schichten bilden, die hydrophile Eigenschaften haben, alternativ aus den aus Cellulose oder aus Acryl- und Metacrylsäure erhaltenen Polymeren ausgewählt sind.

8. Verfahren nach Anspruch 7, dadurch gekennzeichnet, daß die Stoffe, welche die Schicht oder die Schichten mit hydrophilen Eigenschaften bilden, in Gegenwart von Plastifizierungsmitteln verwendet werden.

9. Verfahren nach Anspruch 4, dadurch gekennzeichnet, daß die Stoffe, welche die Schicht oder die Schichten mit lipophilem Charakter bilden, aus Lipiden wie Fettsäuremono-, di- oder triglyceriden, Wachsen, Fettalkoholen und Fettsäuren ausgewählt sind.

10. Verfahren nach den Ansprüchen 1, 4 und 7 bis 9, dadurch gekennzeichnet, daß die Schichten mit lipophilen und hydrophilen Eigenschaften in alternierender Folge verwendet werden, wobei deren Folge und Anzahl von den Diffusions- und physikochemischen Eigenschaften des ausgewählten Wirkstoffes abhängen.

11. Verfahren nach den Ansprüchen 1 bis 10 , dadurch gekennzeichnet, daß die beschichteten Mikrogranulate eine Größe zwischen 50 und 500 $\mu$m aufweisen.

**12.** Verfahren nach den Ansprüchen 1 bis 10, dadurch gekennzeichnet, daß die beschichteten Mikrogranulate eine Größe zwischen 125 und 250 $\mu$m aufweisen.

**13.** Verfahren nach Anspruch 1, dadurch gekennzeichnet, daß der Träger c) neben der sofort verfügbaren Dosis des Wirkstoffes suspendierende, strukturgebende, süßende, puffernde, konservierende und aromatisierende Substanzen enthält.

**14.** Verfahren, dadurch gekennzeichnet, daß die Mischung aus den Komponenten des wie in einem der Ansprüche 1 bis 12 beanspruchten Systems in Wasser oder in einer Mischung aus Wasser und wassermischbaren Lösemitteln suspendiert wird, um eine flüssige Dosis in einer sofort verwendbaren Formulierung zu ergeben.

**15.** Verfahren nach Anspruch 14, dadurch gekennzeichnet, daß die Suspension im flüssigen Träger über einen Zeitraum von mehreren Monaten die Freisetzungseigenschaften der beschichteten Mikrogranulate konstant hält.

**16.** Verfahren nach den Ansprüchen 1 bis 13, dadurch gekennzeichnet, daß die Mischung der Systemkomponenten aus einer extemporanen Zubereitung besteht, welche dann in einem wäßrigen Lösemittel zum Zeitpunkt der therapeutischen Verwendung suspendiert werden kann.

**17.** Verfahren nach Anspruch 16, dadurch gekennzeichnet, daß die durch eine feste Mischung, welche extemporan suspendiert werden kann, bewirkte Formulierung insbesondere für solche Wirkstoffe verwendet wird, welche in wäßrigem Medium eine geringe Stabilität aufweisen.

**18.** Verfahren nach den Ansprüchen 1 bis 17, dadurch gekennzeichnet, daß dieses nach vorhergehender Einstellung der Bestandteile für die Verabreichung und Dosierung von Wirkstoffen geeignet ist, welche sauren, basischen oder neutralen Charakter haben.

**Revendications**

**Revendications pour les Etats contractants suivants : AT, BE, CH, DE, DK, FR, GB, IT, LI, LU, NL, SE**

**1.** Système thérapeutique de formulation de formes pharmaceutiques liquides à libération contrôlée consistant en :
a) microgranulés contenant le principe actif et des excipients appropriés, ayant une taille qui permette aisément leur suspension dans un milieu liquide après avoir été recouverts de manière adéquate de matières polymères.
b) une série de revêtements multicouches à base de polymères lipidiques et de pseudo cire enrobant ces microgranulés tout en maintenant leur taille, de façon à permettre la libération contrôlée du principe actif qu'ils contiennent en périodes qui peuvent être prédéterminées, et à assurer d'une part une dispersion totale des microgranulés enrobés dans le véhicule liquide et d'autre part la conservation des caractéristiques retard pendant une longue période de temps.
c) un véhicule d'aministration liquide pour ces microgranulés enrobés, caractérisé en ce qu'il contient, en plus des additifs appropriés, une quantité immédiatement disponible du même principe actif que celui contenu dans les microgranulés à effet retard.

**2.** Système thérapeutique selon la Revendication 1, caractérisé en ce que le principe actif utilisé est de la théophylline et/ou ses sels pharmaceutiquement acceptables.

**3.** Système thérapeutique selon la Revendication 1, caractérisé en ce que les microgranulés en a) possèdent une teneur importante en principe actif, une surface uniforme, une forme presque sphérique, une densité apparente comprise entre 300 et 800 g/l et une friabilité faible.

**4.** Système thérapeutique selon la Revendication 1, caractérisé en ce que la série d'enrobages mentionnée en b) consiste en une première couche sur la surface des microgranulés décrits en a) ayant pour but de former une barrière insensible aux varations de pH, ce qui permettra un contrôle de la libération du principe actif à l'intérieur des limites de tailles prédéterminées, et consiste en des couches successives posées l'une sur l'autre, constituées en alternance de couches hydrophiles et lipophiles de telle sorte que, en fonction de la nature et des exigences thérapeutiques du principe actif, elles régulent en plus l'effet retard du principe actif apportant en même temps aux granulés enrobés la faculté de se disperser dans le véhicule liquide.

**5.** Système thérapeutique selon la Revendication 4, caractérisé en ce que la première couche de matériau disposée sur le noyau du microgranulé consiste en un mélange, dans des proportions variables, d'une premier composé choisi parmi les dérivés de la cellulose ou de l'acide acrylique ou métacrylique, d'un second composé consistant en huiles végétales hydrogénées ou partiellement hydrogénées et de matériaux plastifiants appropriés .

**6.** Système thérapeutique selon la Revendication 5, caractérisé en ce que le mélange des deux composés est réalisé selon un rapport variant réciproquement entre 20 et 80%.

**7.** Système thérapeutique selon la Revendication 4, caractérisé en ce que les matériaux constituant les couches ou les couches ayant une nature hydrophile sont choisis alternativement parmi des polymères dérivés de la cellulose et des acides acrylique et métacrylique.

**8.** Système selon la revendication 7, caractérisé en ce que les matériaux constituant la couche ou les couches ayant une nature hydrophile sont utilisé en présence de plastifiants.

**9.** Système thérapeutique selon la revendication 4, caractérisé en ce que les matériaux constituant la couche ou les couches ayant un caractère lipophile sont choisis parmi des lipides tels que l'acide gras mono-, di-, tri-glycéride, des cires, des alcools gras, des acides gras.

**10.** Système thérapeutique selon les Revendications 1, 4 et 7 à 9, caractérisé en ce que les couches ayant un caractère lipophile et hydrophile sont posées en alternance, leur succession et leur nombre dépendant des caractéristiques physico-chimiques et de diffusion du principe actif choisi.

**11.** Système thérapeutique selon les Revendications 1 à 10, caractérisé en ce que les microgranulés enrobés ont une taille comprise entre 50 et 500 $\mu$m.

**12.** Système thérapeutique selon les Revendications 1 à 10, caractérisé en ce que les microgranulés enrobés ont une taille comprise entre 125 et 250 $\mu$m.

**13.** Système thérapeutique selon la Revendication 1, caractérisé en ce que le support c) contient outre une dose de principe actif immédiatement disponible : des substances de suspension, de structuration, édulcorantes, tampon, de conservation et aromatisantes.

**14.** Système thérapeutique caractérisé en ce que le mélange de composants du système tel que revendiqué dans l'une quelconque des Revendications 1 à 12, est mis en suspension dans de l'eau ou dans un mélange d'eau et de solvants miscibles dans l'eau, pour obtenir une formule de dosage liquide immédiatement utilisable.

**15.** Système thérapeutique selon la Revendication 14, caractérisé en ce que la suspension maintient constante, dans le milieu liquide, pendant une période de plusieurs mois, les caractéristiques retard des microgranulés enrobés.

**16.** Système thérapeutique selon les Revendication 1 à 13, caractérisé en ce que le mélange des composants du système peut être une formule instantanée que l'on peut mettre en suspension dans un solvant aqueux au moment de son utilisation thérapeutique.

**17.** Système thérapeutique selon la Revendication 16, caractérisé en ce que la formulation, constituée d'un mélange solide qui peut être mis dans une suspension au moment de son utilisation, peut être utilisée en particulier pour des principes actifs ayant une faible stabilité dans un milieu aqueux.

**18.** Système thérapeutique selon les Revendications 1 à 17, caractérisé en ce qu'on peut l'appliquer, après avoir préalablement ajusté ses composants, pour l'administration et le dosage des principes actifs ayant un caractère acide, alcalin ou neutre.

**Revendications pour les Etats contractants suivants : ES, GR**

1. Procédé de préparation d'un système thérapeutique de formulation de formes pharmaceutiques liquides à libération contrôlée consistant en :

   a) microgranulés contenant le principe actif et des excipients appropriés, ayant une taille qui permette aisément leur suspension dans un milieu liquide après avoir été recouverts de manière adéquate de matières polymères.

   b) une série de revêtements multicouches à base de polymères lipidiques et de pseudo cire enrobant ces microgranulés tout en maintenant leur taille, de façon à permettre la libération contrôlée du principe actif qu'ils contiennent en périodes qui peuvent être prédéterminées, et à assurer d'une part une dispersion totale des microgranulés enrobés dans le véhicule liquide et d'autre part la conservation des caractéristiques retard pendant une longue période de temps.

   c) un véhicule d'aministration liquide pour ces microgranulés enrobés, caractérisé en ce qu'il contient, en plus des additifs appropriés, une quantité immédiatement disponible du même principe actif que celui contenu dans les microgranulés à effet retard, et comprenant les étapes suivantes :

      i) le principe actif est tout d'abord mis sous forme de granulés en le malaxant sous mouillage avec les excipients;

      ii) les microgranulés sont enrobés selon la série d'enrobages mentionnée plus haut;

      iii) les granulés enrobés sont introduits dans le véhicule liquide;

      iv) la quantité disponible de principe actif est solubilisée ou introduite dans la forme liquide au moment où on le désire.

2. Procédé selon la Revendication 1, caractérisé en ce que le principe actif utilisé est de la théophylline et/ou ses sels pharmaceutiquement acceptables.

3. Procédé selon la Revendication 1, caractérisé en ce que les microgranulés en a) possèdent une teneur importante en principe actif, une surface uniforme, une forme presque sphérique, une densité apparente comprise entre 300 et 800 g/l et une friabilité faible.

4. Procédé selon la Revendication 1, caractérisé en ce que la série d'enrobages mentionnée en b) consiste en une première couche sur la surface des microgranulés décrits en a) ayant pour but de former une barrière insensible aux varations de pH, ce qui permet un contrôle de la libération du principe actif à l'intérieur des limites de tailles prédéterminées, et consiste en des couches successives posées en succession l'une sur l'autre, constituées en alternance de couches hydrophiles et lipophiles de telle sorte que, en fonction de la nature et des exigences thérapeutiques du principe actif, elles régulent en plus l'effet retard du principe actif apportant en même temps aux granulés enrobés la faculté de se disperser dans le véhicule liquide.

5. Procédé selon la Revendication 4, caractérisé en ce que la première couche de matériau disposée sur le noyau du microgranulé consiste en un mélange, dans des proportions variables, d'un premier composé choisi parmi les dérivés de la cellulose ou de l'acide acrylique ou métacrylique, d'un second composé consistant en huiles végétales hydrogénées ou partiellement hydrogénées et de matériaux plastifiants appropriés .

6. Procédé selon la Revendication 5, caractérisé en ce que le mélange des deux composés est réalisé selon un rapport variant réciproquement entre 20 et 80%

7. Procédé selon la Revendication 4, caractérisé en ce que les matériaux constituant la couche ou les couches ayant une nature hydrophile sont choisis alternativement parmi des polymères dérivés de la cellulose et des acides acrylique et métacrylique.

8. Procédé selon la revendication 7, caractérisé en ce que les matériaux constituant la couche ou les couches ayant une nature hydrophile sont utilisé en présence de plastifiants.

9. Procédé thérapeutique selon la revendication 4, caractérisé en ce que les matériaux constituant la couche ou les couches ayant un caractère lipophile sont choisis parmi des lipides tels que l'acide gras mono-, di-, tri-glycéride, des cires, des alcools gras, des acides gras.

**10.** Procédé selon les Revendications 1, 4 et 7 à 9, caractérisé en ce que les couches ayant un caractère lipophile et hydrophile sont posées en alternance leur succession et leur nombre dépendant des caractéristiques physico-chimiques et de diffusion du principe actif choisi.

**11.** Procédé selon les Revendications 1 à 10, caractérisé en ce que les microgranulés enrobés ont une taille comprise entre 50 et 500 $\mu$m.

**12.** Procédé selon les Revendications 1 à 10, caractérisé en ce que les microgranulés enrobés ont une taille comprise entre 125 et 250 $\mu$m.

**13.** Procédé selon la Revendication 1, caractérisé en ce que le support c) contient outre une dose de principe actif immédiatement disponible : des substances de suspension, de structuration, édulcorantes, tampon, de conservation et aromatisantes.

**14.** Procédé caractérisé en ce que le mélange constitué par les composants du système tel que revendiqué dans l'une quelconque des Revendications 1 à 12, est mis en suspension dans de l'eau ou dans un mélange d'eau et de solvants miscibles dans l'eau, pour obtenir une formule de dosage liquide immédiatement utilisable.

**15.** Procédé selon la Revendication 14, caractérisé en ce que la suspension maintient constante, dans le milieu liquide, pendant une période de plusieurs mois, les caractéristiques retard des microgranulés enrobés.

**16.** Procédé selon les Revendication 1 à 13, caractérisé en ce que le mélange des composants du système peut être une formule instantanée que l'on peut mettre en suspension dans un solvant aqueux au moment de son utilisation thérapeutique.

**17.** Procédé selon la Revendication 16, caractérisé en ce que la formulation, constituée d'un mélange solide qui peut être mis dans une suspension au moment de son utilisation, peut être utilisée en particulier pour des principes actifs ayant une faible stabilité dans un milieu aqueux.

**18.** Procédé selon les Revendications 1 à 17, caractérisé en ce qu'on peut l'appliquer, après avoir préalablement ajusté ses composants, pour l'administration et le dosage des principes actifs ayant un caractère acide, alcalin ou neutre.